(19) Europäisches Patentamt
European Patent Office
Office européen des brevets

(11) **EP 4 446 093 A1**

(12) **EUROPEAN PATENT APPLICATION**
published in accordance with Art. 153(4) EPC

(43) Date of publication:
**16.10.2024 Bulletin 2024/42**

(21) Application number: **22904619.8**

(22) Date of filing: **06.12.2022**

(51) International Patent Classification (IPC):
**B29C 64/209** (2017.01)       **C12M 1/00** (2006.01)
**B29C 64/321** (2017.01)       **B29C 48/30** (2019.01)
**B29C 48/40** (2019.01)        **B29C 48/285** (2019.01)
**B29C 48/68** (2019.01)        **B33Y 30/00** (2015.01)
**B33Y 40/00** (2020.01)        **B33Y 70/00** (2020.01)

(52) Cooperative Patent Classification (CPC):
**B29C 48/285; B29C 48/30; B29C 48/40;**
**B29C 48/68; B29C 64/209; B29C 64/321;**
**B33Y 30/00; B33Y 40/00; B33Y 70/00; C12M 1/00**

(86) International application number:
**PCT/KR2022/019702**

(87) International publication number:
**WO 2023/106789 (15.06.2023 Gazette 2023/24)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **06.12.2021  KR 20210172761**
**16.08.2022  KR 20220102240**

(71) Applicants:
• **Matrixcell Bio Co., Ltd.**
**Seoul 01811 (KR)**

• **Foundation For Research And Business,**
**Seoul National University of Science and**
**Technology**
**Seoul 01811 (KR)**

(72) Inventors:
• **NOH, Insup**
**Seoul 01727 (KR)**
• **BHATTACHARYYA, Amitava**
**Seoul 01852 (KR)**

(74) Representative: **Grünecker Patent- und**
**Rechtsanwälte**
**PartG mbB**
**Leopoldstraße 4**
**80802 München (DE)**

(54) **BIO-PEN STRUCTURE FOR IMPROVING MIXING HOMOGENEITY, AND BIO-PRINTING MEHOD USING SAME**

(57)    Disclosed in the present application are: a pen-type structure for mixing and discharging a bio-ink or a hydrogel: and a bio-ink or hydrogel printing method using same. The pen-type structure comprises: a cylindrical first barrel for housing a first screw; a cylindrical second barrel for housing a second screw, which is longer than the first screw and has a structure parallel with the first screw; a controller connected to a gear of the first screw and a gear of the second screw so as to drive the first screw and the second screw; two or more supply units formed in the first barrier and supplying a bio-ink or hydrogel material into the first barrel; and a bio-ink or hydrogel discharge unit which extends from the end portion of the second barrel on the opposite side of the controller, and which discharges bio-ink or hydrogel.

Fig. 3

UV irradiator for light cross-linkage
Reinforced barrel

Electric wire connected to UV device

UV irradiation device

Electric wire - main frame connected

Electric wire barrel for producing light irradiation gel

Solidifying gel

Inlet 1 (for biomaterials)

Barrel

83.5

Short screw

Inlet 2 (for live cells)

Roller head for large area printing (detachable)

26.7

Long screw

100

111

## Description

[Technical Field]

The present application discloses a pen-type structure for mixing and discharging bio-ink or hydrogel, and a method for mixing and printing bio-ink or hydrogel using the same.

[Background Art]

Bio-ink printing is applied to tissue engineering tissue regeneration through technologies such as extrusion type, bio-ink type, and laser-assisted printing (stereolithography apparatus: SLA). The most widely used method is an extrusion-type 3D bio-printing technology. In the case of cell-containing 3D bio-printing and tissue engineering regenerative medicine, hydrogel (hydrogel in the case of not mixing cells, and bio-ink in the case of including cells) including multiple components such as cells, nanoparticles, growth factors, etc., is used to overcome the limits of printing with a single component such as cells. In order to uniformly distribute various components including gel, biomolecules, and bioactive micro/nanoparticles within hydrogel (bio-ink in the case of containing cells), the conventional technology requires several preparation steps such as injecting various components into beakers, tubes, etc., respectively, mixing the same using mechanical and manual methods such as stirrer, pipetting, spatula, centrifugation, syringe, etc., (such as mixing in the process of injecting each solution into two parallel or independent barrels) and the like. In the process of mixing cells through the procedures of the various preparation steps, bio-ink is prepared in a high shear mixing process by mixing live cells in hydrogel before the 3D bio-printing process. The bio-ink prepared through the above process is loaded into an extruder head of a 3D bio-printer, and extruded to prepare a mimic structure (tissue regeneration structure) of tissues and organs of the human body, and then the tissue engineering structure is subjected to *in vitro* cell culture to regenerate tissues (*in vitro* tissue regeneration), implant *in vitro* regeneration tissues into defective tissues of the human body, such as cartilage, skin, bone, nerve, spine, etc., thereby inducing regeneration of tissues (*in vivo* tissue regeneration), or make a direct injection, thereby treating damaged tissues or inducing tissue regeneration (cell therapeutic agent). At this time, only inanimate matters such as growth factors, genes, peptides, drugs, particles, etc., are included in hydrogel without containing cells, which are then used as a bioactive material carrier, and thus utilized in bone filler in dental clinics or for inducing regeneration of defective tissues (gene therapeutic agent, cell therapeutic agent, etc.).

However, the currently used bio-ink printing technique and the bioactive material-containing hydrogel preparation technique are problematic in that cells are not uniformly dispersed in the bio-ink and the bioactive material is not well dispersed in the hydrogel because the components are induced to be manually mixed, the bio-ink in which cells, growth factors, and the like are not homogenously dispersed is manually loaded into an extruder head and used, or the cells and the hydrogel are mixed by putting the cells and the hydrogel into two separate syringe barrels and then extruding the same. Such bio-printing result causes a problem in that the physical properties of the tissue to be finally regenerated may not be controlled (e.g., a problem in that tissue regeneration becomes non-uniform due to non-uniform dispersion of cells in the structure). In addition, the problem in which tissue regeneration by a biodegradable tissue engineering structure is non-uniformly induced (for example, tissue regeneration becomes non-uniform due to non-uniform dispersion of cells and growth factors) is caused due to the problems in which it is difficult to extrude high-viscosity gel in an air pressure or piston-type extrusion system, cells loaded in high-viscosity bio-ink are damaged during extrusion, and cells and bioactive factors (growth factors, nanoparticles, etc.) are not uniformly distributed in a tissue engineering structure (scaffold).

Furthermore, in the case of cell therapeutic agents for treating diseased tissues through an injection type of including cells/stem cells in hydrogel, the cells are not homogenously delivered to damaged tissues, and thus tissue treatment is not efficiently performed.

Moreover, in the case of drug carriers or gene therapeutic agents for treating diseased tissues through an injection type of including cells/stem cells in hydrogel, drugs and genes are not homogenously delivered to damaged tissues, and thus tissue treatment is not efficiently performed.

Besides, even in the case of using bioactive materials of inanimate matters only such as bone fillers, there is a problem in that bioactive material particles such as drugs, growth factors (BMP2, FGF, VEGF, etc.), nanoparticles, etc., are not uniformly mixed in the hydrogel. Thus, after the bioactive material carrier is implanted, the bioactive materials are not homogeneously distributed in defective tissues of patients, and thus bone tissue regeneration are carried out in a different manner along with non-uniform attachment of cells, thus failing to achieve original purpose of homogeneously regenerating defective tissues and treating diseased tissues.

In addition, in the case of tissue reconstruction surgery, medical professionals use hydrogel or bio-ink containing cells for a purpose of using as a cell therapeutic agent. Such cell therapeutic agent requires a preparation process and techniques with complicated steps in the preparation process, and also a damage site, a degree of damage, an amount of cell therapeutic agent/ink required for each patient, a concentration of cell therapeutic agent/ink concentration, a shape of cell therapeutic agent/ink shape, etc., are differently applied for each patient. Thus, it is difficult to achieve uniform mixing of cells and bioactive materials with conventional methods using the same process in the preparation process. In addition, considering a situation in which a surgeon needs to directly add a drug or a bioactive factor to hydrogel as needed and apply the resulting product in the field, it is not easy for the surgeon to uniformly mix cells and bioactive materials when mixing in the field. Even when a prepared cell therapeutic agent is supplied and used, there is a problem in that cells, growth factors, etc., are precipitated while the prepared cell therapeutic agent is stored or frozen and thus are not uniformly mixed at a time to be used, and there is also a need, etc., for a thawing process in the case of freezing, thus resulting in the inability to induce homogeneous tissue regeneration and cell therapy effects, which are the original purpose, and urging a need to use a mixture of biomaterials and hydrogel directly in the field.

In addition, a non-uniformity problem of cells and bioactive materials in bio-ink occurs similarly in research sites of universities and research institutes. There is a problem in deriving experimental results by uniform dispersion in an experiment on bio-ink and bioactive material carriers containing cells or bioactive materials. Since different mixtures are prepared according to the skill of researchers, and the mixing process is manually performed, there is a disadvantage in that the bio-ink mixed components are not stable (in other words, not standardized).

Further, most stem cells are used by being injected into a buffer solution, a biocompatible polymer solution, or hydrogel, and high density/expensive stem cells are used in order to ensure high viability and activity of cells, which requires high costs. Moreover, in the case of skin tissue regeneration surgery having a large reconstruction area, the problem that a printing line width is not wide upon bio-ink printing according to the related art is not solved. In a process of printing a wide area, uniform distribution of cells is important, and in particular, it is very important to ensure a printing line of printing inks and a continuous connection of lines, along with cell uniformity between layers (horizontal and vertical connections).

In order to prepare a 3D bio-printing tissue engineering structure (scaffold) suitable for a wide range of bio-ink types and patient characteristics, various types of bio-inks, ink mixing devices, mixing systems of cell therapy carriers, and 3D bio-printing extrusion heads have been proposed in order to solve disadvantages of extrusion heads as well as several steps of preparation processes.

As part of the development of bio-printing extrusion heads, Korean Registered Patent No. 10-2286073 discloses a dual screw extrusion mixing system including two parallel screw extruders having the same length. However, the invention had a limitation in the semi-automatic and continuous loading of bio-ink into the extruder while uniformly mixing the bio-ink by the parallel screw extruder technique, and it was difficult to print the bio-ink with a continuous and constant line width because bio-printing is performed with a predetermined capacity of ink using two parallel screws having the same length. In addition, due to a structural limitation in the design of the extruder, it was difficult to print with high precision, and it was difficult to uniformly print a large area such as skin, film, and wound dressing. In addition, there was a problem in that there is no function of converting a polymer into gel while mixing the polymer with a solution, the shaped hydrogel is not converted into a nanoparticle gel, and the encapsulation of drugs is not efficient. Further, the conventional extruder mounted on the bioprinter was not be independently used, and thus had a problem in that it needs to be mounted on the bioprinter and used, and had a problem in that it is impossible to directly print on the tissue of a patient (e.g., skin, cartilage, etc.), and a problem in that the standards of a tissue engineering support to be manufactured are limited to a printing range of the 3D printer.

[Disclosure]

[Technical Problem]

Accordingly, to solve the conventional problems described above, the present inventors have made it possible to mix bio-ink or hydrogel in an extruder and control a printing speed, in which the extruder is composed of screws having different lengths, a part of which is composed of two parallel screws, and a controller is connected. The extruder may be designed to be driven with two screws having different lengths, so that gel and cells injected through different inlets may be mixed in a mixing step of a parallel screw to a later step (but, a step before the mixing with the parallel screw is finished) which is an end step, and a printing speed and an operation time may be adjusted with the controller. In addition, a rotation speed of the screw may be adjusted by using the controller, the screw may be allowed to have a variable pitch including a short pitch (an area with a best mixing power of nanoparticles and gel and a highest probability of cell damage), a medium pitch, and a long pitch (an area with a low mixing power of gel and nanoparticles and a lowest probability of cell damage) from a distance close to the controller toward a bio-ink or hydrogel discharge unit, a mixing performance of gel and nanoparticles may be maximized in a state in which cells are not supplied in the short pitch section, and the cells may be injected in an area with a least cell damage, that is, a long pitch section, thereby minimizing a shear force of the cells and the cell damage. Furthermore, in order to maximize printing properties, an area composed of only a long screw may be designed at a discharged portion, thereby more precisely performing a delivery function of the mixed bio-ink or hydrogel as well as a printing function, and an extrusion head in the form of a brush or roller may be designed to be mounted so as to print over a large area.

The mixing and movement with two screws may be changed into the mixing and movement with one screw in the middle (that is, the two screw operations are merged into one screw operation in the middle), thereby minimizing the damage to cells injected in a later step. Furthermore, the bio-ink or hydrogel may be outputted by one screw (that is, a screw having a longer length) so as to be more uniformly and precisely controlled, and the bio-ink or hydrogel may be uniformly mixed. In order to ensure uniform mixing of bio-ink or hydrogel containing micro/nanomaterials and other components, a semi-automatic twin screw extruder (TSE) head may be designed to simultaneously perform 3D bio-printing and mixing (a syringe containing a sterilized bio-ink mixture is provided to be replaced during a process, thereby enabling continuous printing).

A partially engaged biaxial extrusion-based pen-type structure (hereinafter, also referred to as a bio-pen) according to the present invention may minimize cell damage by using a reverse rotation (reverse pitch) in a top-down approach, adjust a distance between an end of a short screw and a barrel to minimize damage to bio-ink components (a mixture of cells, nanoparticles, growth factors, and the like), and directly load a drug into gel to encapsulate the drug in the gel and prepare a nanoparticulate gel. In addition, screws may be simplified into one screw in an end portion from which the content is discharged so as to reduce a load of a driving motor, a diameter of a nozzle in the discharge unit may be gradually reduced to increase a printing precision, and a bio-pen capable of mixing and printing bio-ink or hydrogel may be detached from a printer to enable movement and direct printing by hand.

Furthermore, a polymer solution may be converted into hydrogel during the printing process, and the printing may be laminated to prepare a structure. In order to chemically crosslink a polymer solution including a photoinitiator and convert into gel, a UV LED providing unit capable of inducing an activity of the photoinitiator may be installed on a barrel adjacent to the bio-ink or hydrogel discharge unit. An UV LED installation unit may be designed to install a passage and components capable of installing an electric wire connected to upper and lower portions of the barrel on the barrel, and a device capable of turning on/off UV light may be configured in a controller of an upper portion of the barrel.

In addition, during the printing process, it may be possible to prepare a self-bonding hydrogel (e.g., fibrin gel, Diel-Alders reaction, thiol-ene reaction, Michael addition reaction, etc.) without using a photoinitiator. In other words, two types of polymer precursor solution may be simultaneously injected into an upper inlet and mixed to induce gel, or injected into the upper and lower inlets, respectively, and uniformly mixed to enable synthesis of hydrogel in which a homogeneous polymer network is formed (e.g., fibrin gel).

Furthermore, a driving unit capable of controlling a rotation speed and driving time of the screw, that is, a controller, may be provided at an upper portion of the barrel, and an inlet capable of injecting gel at an upper portion of the barrel and an inlet for injecting cells at a lower portion of the barrel may be provided, so that the bio-pen detached from a 3D printer may become a bio-printing system which enables direct printing by hand and thus may be used as an independent device. The driving unit may be equipped with a display capable of adjusting a rotation speed and a driving time and visually checking the rotation speed and driving time of the driving unit. A battery may be mounted as a driving device so that the bio-pen may be independently driven, and the bio-pen may be designed to be connected to an electric adapter and a computer for a long stable use, so that the bio-pen may be stably driven. In addition, a mobile bio-pen capable of hand-printing/hand-writing may enable free printing on a large-area surface, and may enable free printing by hand, thus providing an advantage that bio-ink or hydrogel may be applied to a large-area surface such as damaged skin. To this end, a roller or a brush may be provided as an extrusion head to the bio-ink or hydrogel discharge unit, so that the bio-ink or hydrogel may be applied by being printed horizontally or vertically in a laminated manner and the non-uniformity caused between printing lines may be minimized.

Meanwhile, the bio-ink containing cells/stem cells may be injected directly or indirectly (for example, uniformly mixing the bio-ink with a bio-pen and then transferring the uniformly mixed bio-ink to a syringe) into damaged tissues such as cartilage, tendon, bone, blood vessel, eye, skin, or the like using a mobile bio-pen, and thus may provide a function of mixing cell therapy components and using as a cell therapy carrier. In addition, there may be provided as a function of delivering a uniform mixture of various bioactive materials (drugs, nanoparticles, growth factors, ceramic particles, bone graft materials, etc.) which do not contain cells, thereby promoting tissue regeneration of a defective site or treating a damaged site.

As another use of the bio-pen according to the present invention, it may be used as a device in which a pre-formed hydrogel is provided at an inlet on an upper portion thereof, and drugs or bioactive materials having various molecular weights are provided at an inlet on a lower portion thereof, thereby effectively loading, into the hydrogel, drugs having low molecular weights (e.g., oligopeptides, etc.) and bioactive materials having high molecular weights (e.g., fucoidan, hyaluronic acid, albumin, protein drugs, etc.) in a homogeneous physical manner.

The bio-pen according to the present invention may be remotely controlled by being connected to Wi-Fi, Bluetooth, and a computer, may be stably used by being fixed at a clean bench of a laboratory, and may be stably provided with power by connecting an adapter to the bio-pen through an electrical outlet.

In one aspect, an object of the present invention is to provide a pen-type structure for mixing and discharging bio-ink or hydrogel.

In another aspect, an object of the present invention is to provide a bio-ink or hydrogel printing method using the pen-type structure for mixing and discharging the bio-ink or the hydrogel.

In still another aspect, an object of the present invention is to provide a tissue engineering structure using the pen-type printing system.

In still another aspect, an object of the present invention is to provide a bio-ink or hydrogel printing method capable of preparing a tissue engineering structure using the pen-type printing system.

[Technical Solution]

In one aspect, the present invention may provide a pen-type structure for mixing and discharging bio-ink or hydrogel including: a cylindrical first barrel for housing a first screw; a cylindrical second barrel for housing a second screw, which is longer than the first screw and has a structure parallel with the first screw, and formed to have a length longer than that of the first barrel; a controller connected to a gear of the first screw and a gear of the second screw in adjacent to the gear of the first screw and the gear of the second screw so as to drive the first screw and the second screw; two or more supply units formed in the first barrier and supplying a bio-ink or hydrogel material into the first barrel; and a bio-ink or hydrogel discharge unit which extends from an end portion of the second barrel on an opposite side of the controller, and which discharges bio-ink or hydrogel, in which the first barrel and the second barrel are formed to extend in communication with each other so that the first screw and the second screw are not spatially separated, and in which the first screw has a variable pitch of three sections and the second screw has a variable pitch of four sections.

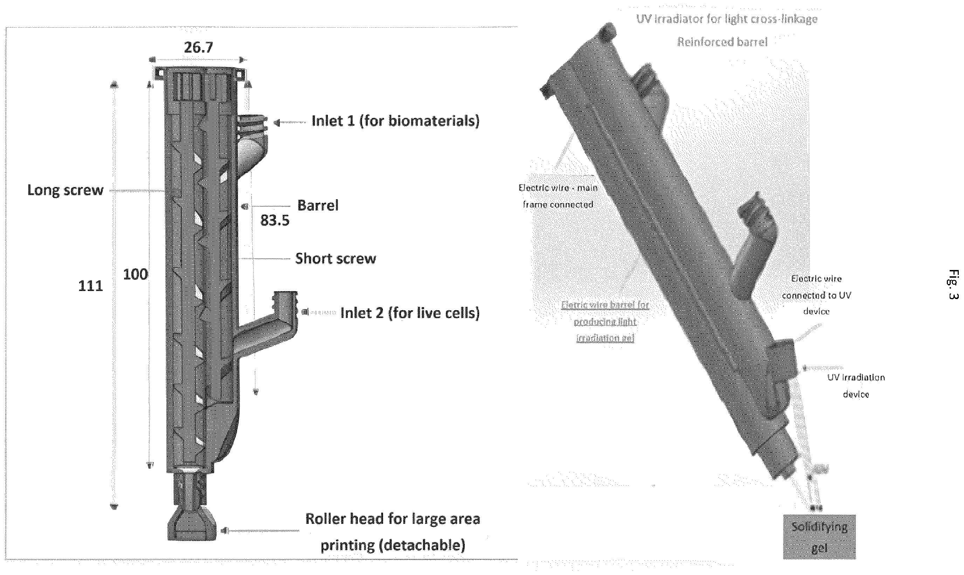

In one exemplary embodiment, a UV irradiation member may be provided at a lower end of the first barrel and/or the second barrel so as to irradiate UV light onto the bio-ink or hydrogel to be discharged, and a passage (i.e., an electric wire tube for preparing a light irradiation gel) for connecting the UV irradiation member and a controller at an upper portion of the barrel may be designed (see FIG. 3). The polymer solution including the photoinitiator provided through the supply unit during the printing process may be irradiated with UV to be cross-linked by the hydrogel, thus providing an effect of laminating and printing the gel. Accordingly, the pen-type structure may be used as a device capable of preparing a 3D bioprinting structure.

In one exemplary embodiment, the first screw (short screw) may have a section a (introduction section), a section b (high gel mixing section), and a section c (cell mixing section) sequentially in a direction from the controller to the bio-ink or hydrogel discharge unit, a pitch size of each section may be $b < a \leq c$, the second screw may have a section d (introduction section), a section e (high gel mixing section), a section f (cell mixing section), and a section g (printing ink delivery section) sequentially in a direction from the controller to the bio-ink or hydrogel discharge unit, a pitch size of each section may be $e <$

$d \leq f$, and a pitch size of the section g may satisfy the following conditions i) and ii):

$$i)\ g < f$$

$$ii)\ e \leq g\ or\ e > g.$$

The sections a, b, and c of the first screw and the sections d, e, and f of the second screw may have the same pitch structure and length, and have only a phase difference.

In one exemplary embodiment, a pitch size of the section d and the section g may be g < d.

In one exemplary embodiment, the first screw and the second screw may have a pitch phase difference of 45° to 135°.

In one exemplary embodiment, a distance between a thread of the first screw and an inner wall of the first barrel may range from 0.005 mm to 0.30 mm, and a distance between a thread of the second screw and an inner wall of the second barrel may range from 0.005 mm to 0.30 mm.

In one exemplary embodiment, a distance between a root of the first screw and an inner wall of the first barrel may range from 0.01 mm to 6 mm, and a distance between a root of the second screw and an inner wall of the second barrel may range from 0.01 mm to 6 mm.

In one exemplary embodiment, a distance between a shaft center of the first screw and a shaft center of the second screw may be formed to be longer than a shaft diameter of the first screw or the second screw.

In one exemplary embodiment, a lower end portion of the first barrel may have an inner wall in a direction perpendicular to an axis of the first screw, and a distance between the inner wall and an end point of the first screw may range from 0.005 mm to 1 mm.

In one exemplary embodiment, the second screw may be formed up to a point where a lower end portion of the second barrel and the bio-ink or hydrogel discharge unit come into contact with each other.

In one exemplary embodiment, an interval of the supply units may not exceed a length corresponding to 1/3 of a length of the second barrel.

In one exemplary embodiment, the bio-ink or hydrogel discharge unit may be capable of attaching and detaching an extrusion head selected from a roller, a brush, or a needle.

In one exemplary embodiment, the bio-ink or hydrogel discharge unit may be a cap which is attached or detached for a use of blocking an outlet of the discharge unit to prepare a nanoparticle gel.

In one exemplary embodiment, the pen-type structure may be mounted to be operable in a printing system.

In one exemplary embodiment, the first barrel and/or the second barrel may include a UV irradiation member for irradiating UV to the bio-ink or hydrogel to be discharged.

In another aspect, the present invention may provide a method for printing bio-ink or hydrogel using the pen-type structure for mixing and discharging the bio-ink or the hydrogel.

In one exemplary embodiment, the method may include preparing nanogel from shaped hydrogel.

[Effects of the Invention]

In one aspect, the present invention may have an effect of providing a pen-type structure for homogeneously mixing and discharging bio-ink or hydrogel.

In another aspect, the present invention may have an effect of providing a pen-type structure for injecting a hydrogel precursor, homogeneously mixing and forming gel to discharge.

In another aspect, the present invention may have an effect of providing a bio-ink or hydrogel printing method using the pen-type structure for mixing and discharging the bio-ink or the hydrogel.

The present invention may have an effect of extruding bio-ink in which nanoparticles and/or living cells are uniformly dispersed, and extruding gels of various viscosities which may not be extruded with conventional pneumatic or piston-type extrusion systems through a precise and positive control, thereby uniformly and finely depositing (laminating) bio-ink or hydrogel through a precise control and continuously and regularly providing gel and/or bio-ink.

The present invention may have an effect of providing a hydrogel drug carrier by physically loading drugs, liposomes, exosomes, and bioactive materials (nanoparticles, proteins, nucleic acids, etc.) into a previously formed hydrogel (e.g., gelatin methacrylate gel, poly (ethylene oxide) gel, hyaluronic acid gel, combucha gel, multi-component polymer gel, and the like) by a rotation shear mechanism.

The present invention may have an effect of preparing a previously formed hydrogel in a nano- and/or microgel particle size and using as a cell therapeutic agent and a drug carrier.

The present invention may have an advantage of preparing an *in situ* 3D printing tissue engineering structure by inducing optical cross-linking while printing (or injecting) a polymer solution mixed with a photoinitiator using a light irradiation system mounted on a bio-pen.

The present invention may have an effect of preparing and providing a tissue engineering structure from bio-ink, hydrogel, biodegradable polymer, etc., by using a fixed and/or mobile bio-pen to be mounted in an existing printer or freely printed by hand.

The present invention may have an effect of providing the homogeneity and the mechanical/biological properties of tissues to be finally regenerated by homogeneously mixing homogeneous drugs, bioactive materials, growth factors, and the like when tissues are regenerated using a tissue engineering structure.

The present invention may enable printing a large area through a horizontal deposition or lamination with a roller or a brush and thus may have an effect of effectively preparing a homogeneous bio-ink or hydrogel printing structure in preparing a tissue regeneration structure such as skin with a large area and multiple layers, cartilage with a complicated shape, brain, etc., a wound dressing, a film, and the like.

In the case of conventional 3D bioprinting, it has been difficult to prepare a structure for a defective site (tissue, organ) having a complicated structure such as cartilage or brain and a structure in which a shape thereof varies depending on a height of layers, but the mobile bio-pen of the present invention may have a ripple effect in which a user may solve the above problems by freely printing bio-ink with the mobile bio-pen.

In addition, the present invention may have an effect of being used as a 3D bio-printing extruder, a mobile printing bio-pen, a cell therapy carrier, and a bioactive material carrier with or without cells by mixing various bio-related solutions, and may have an effect of being applied to regeneration of various tissues or treatment of diseases in musculoskeletal systems including bones, cartilage, spine, nerves, skin, blood vessels, and the like, dental clinic, ophthalmology, circulatory system, brain and the like.

[Description of Drawings]

FIG. 1 shows each component of a pen-type structure according to one embodiment.
FIG. 2 shows a series of processes for assembling and using a pen-type structure according to one embodiment.
FIG. 3 shows a schematic view of a pen-type structure according to one embodiment.

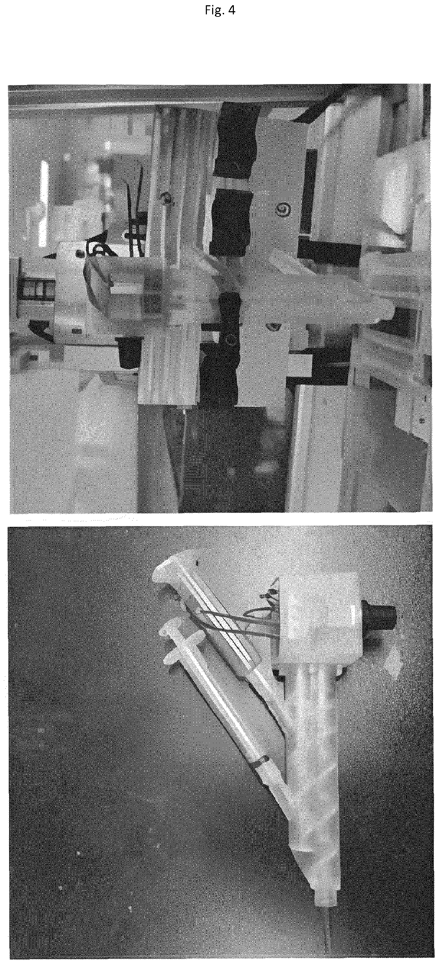

FIG. 4 shows a pen-type structure (left) according to one embodiment and a state in which the pen-type structure is mounted on a printing system (right).

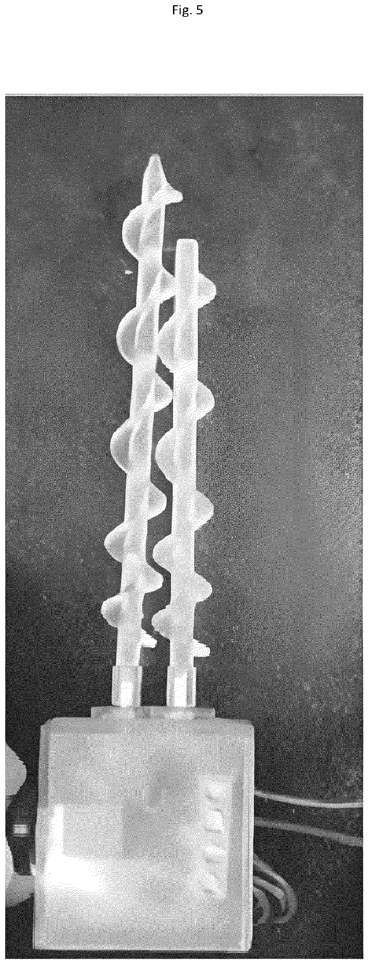

FIG. 5 shows a state in which a barrel is not mounted on a pen-type structure according to one embodiment.

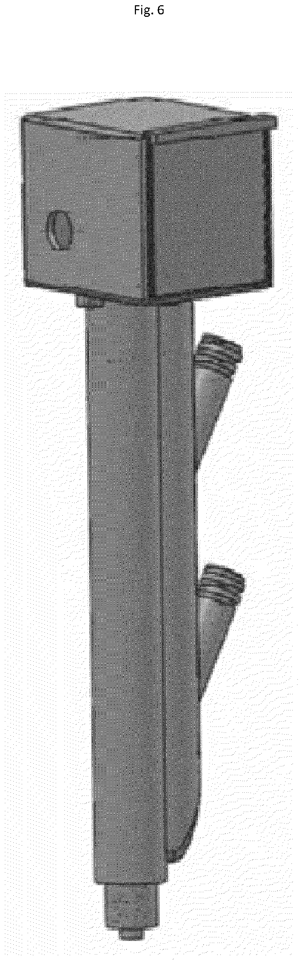

FIG. 6 shows a perspective view of a pen-type structure according to one embodiment.

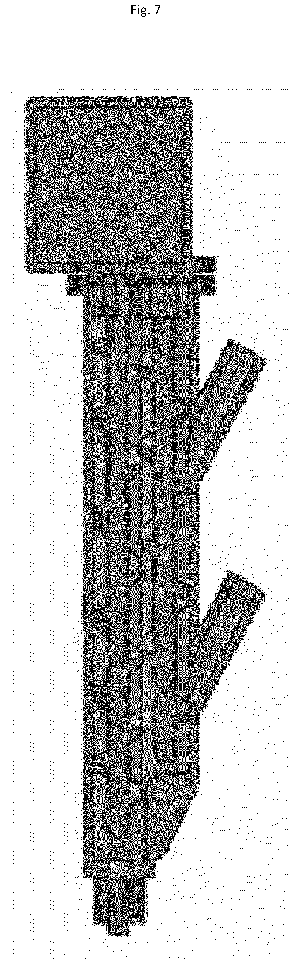

FIG. 7 shows one sectional view of a pen-type structure according to one embodiment.

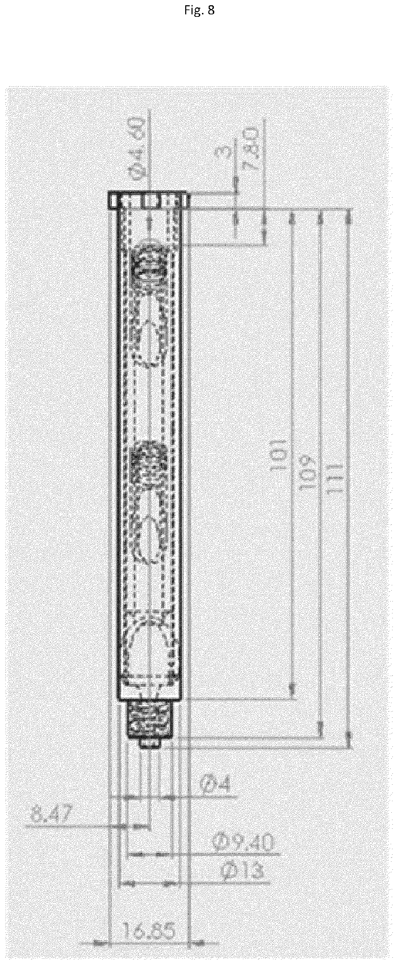

FIG. 8 shows a layout view of one section of a pen-type structure according to one embodiment. In the structure, two supply units which supply a bio-ink or hydrogel material may be located on the same line in a longitudinal direction of a barrel.

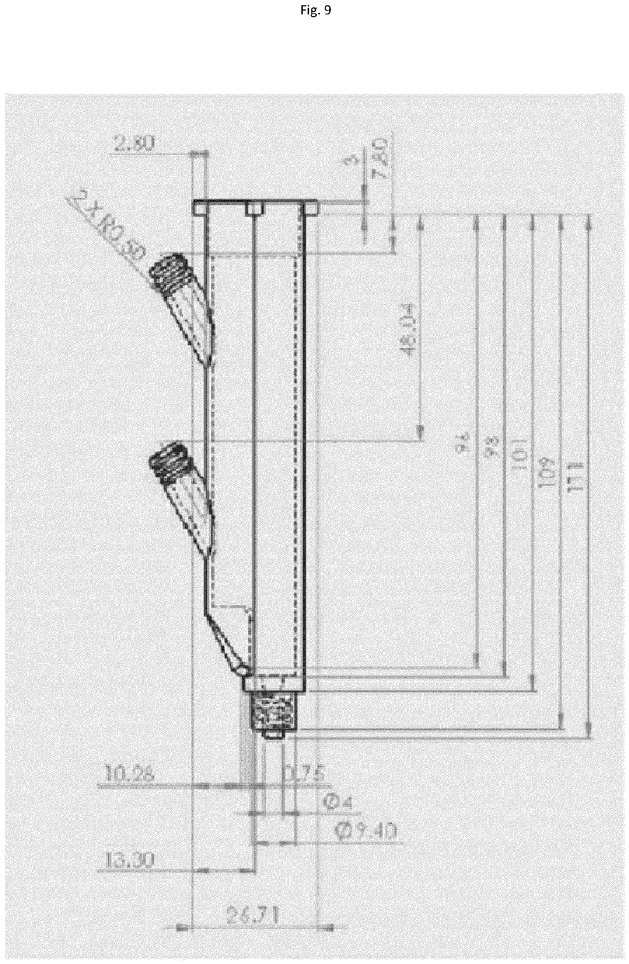

FIG. 9 shows a layout view of one section of a pen-type structure according to one embodiment. In the structure, two supply units which supply a bio-ink material may be located on the same line in a longitudinal direction of a barrel.

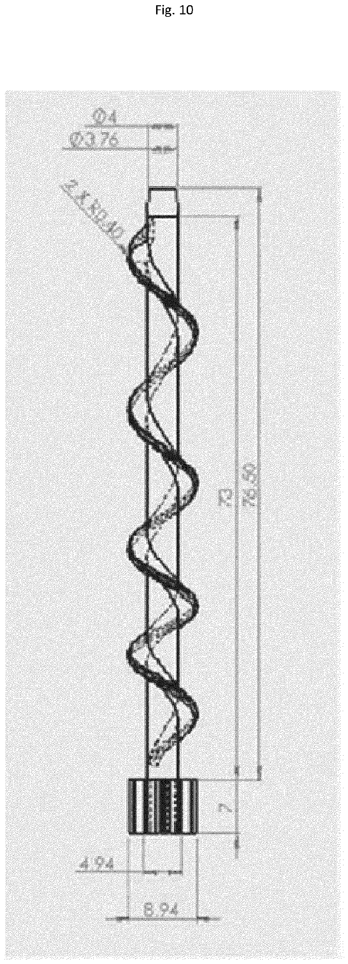

FIG. 10 shows a layout view of a first screw of a pen-type structure according to one embodiment.

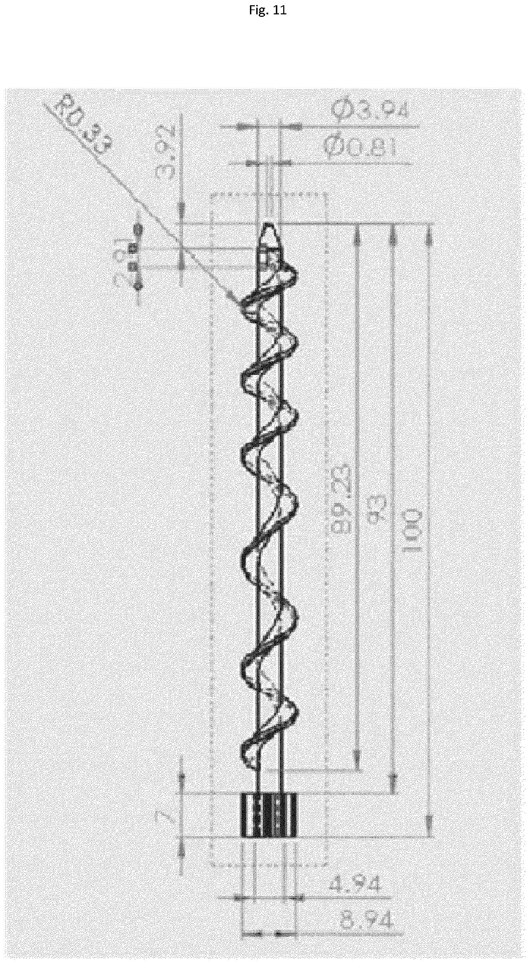

FIG. 11 shows a layout view of a second screw of a pen-type structure according to one embodiment.

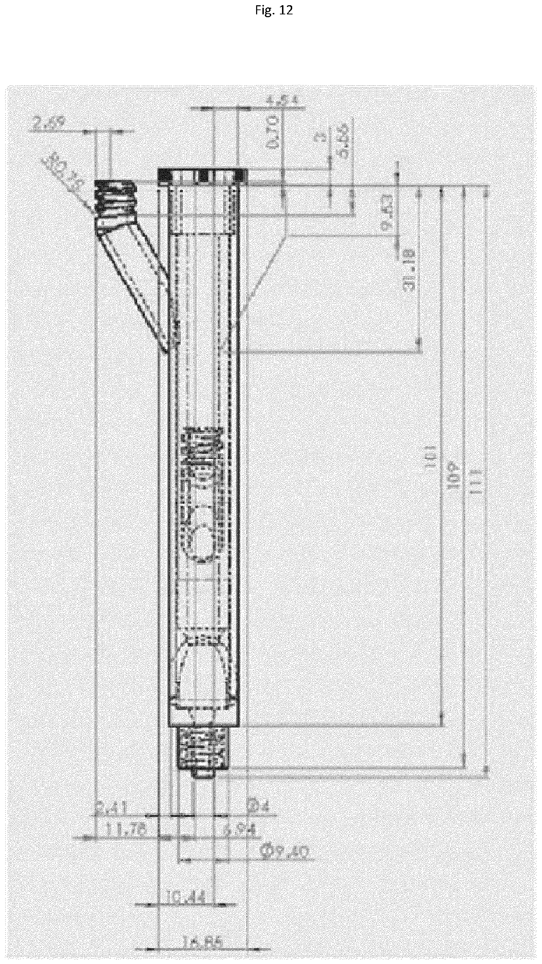

FIG. 12 shows a layout view of one section of a pen-type structure according to one embodiment. In the structure, two supply units which supply a bio-ink or hydrogel material may be located on different lines in a longitudinal direction of a barrel.

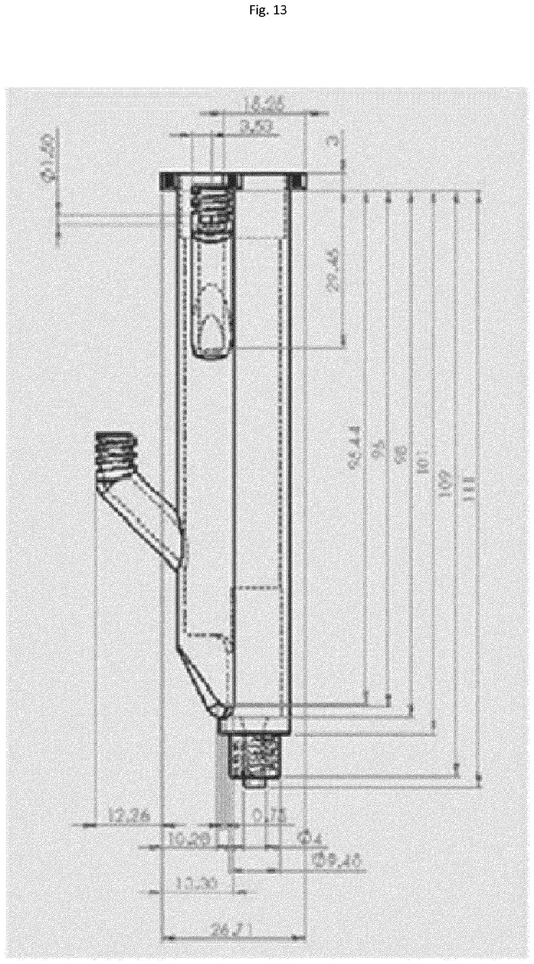

FIG. 13 shows a layout view of one section of a pen-type structure according to one embodiment. In the structure, two supply units which supply a bio-ink or hydrogel material may be located on different lines in a longitudinal direction of a barrel.

FIG. 14 shows one sectional view of a pen-type structure according to one embodiment.

FIG. 15 shows an enlarged view of a distance between an inner wall of a lower end portion of a first barrel of a pen-type structure and an end point of a first screw according to one embodiment.

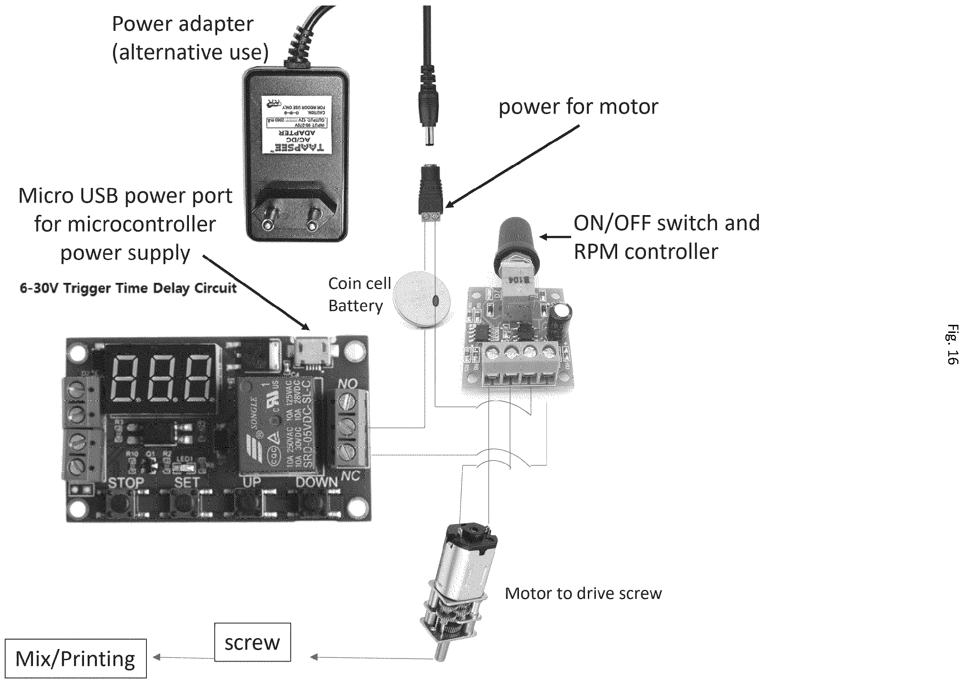

FIG. 16 shows a driving flow of a pen-type structure according to one embodiment.

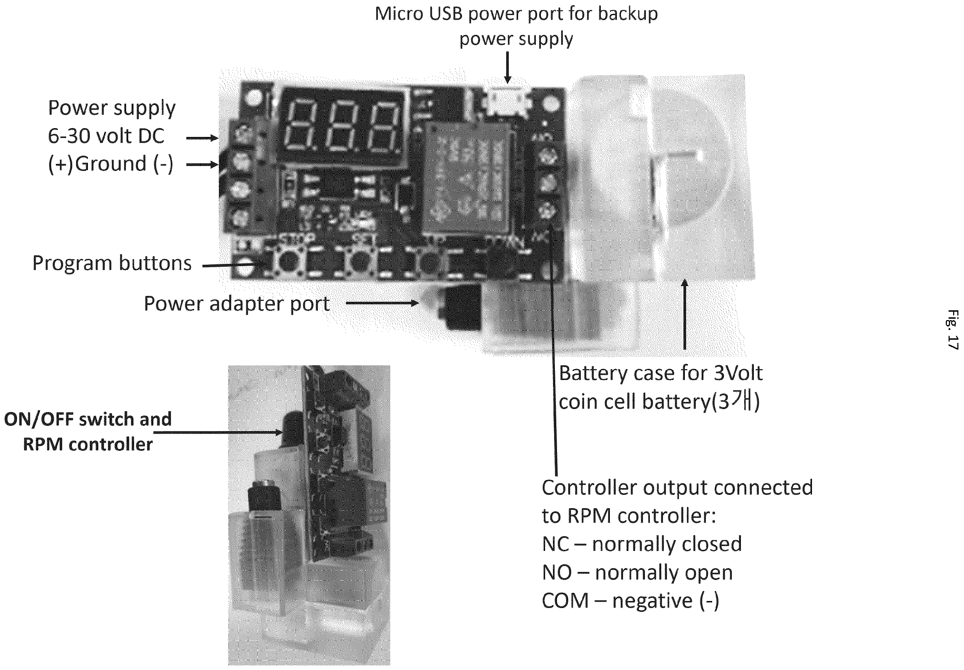

FIG. 17 shows a view of a controller of a pen-type structure according to one embodiment.

FIG. 18 shows a result of culturing cells after mixing bio-ink using a pen-type structure according to one embodiment.

FIG. 19 shows a result of printing using a pen-type structure according to one embodiment.

FIG. 20 shows a schematic view of a process of preparing nano-microgel particles using a pen-type structure according to one embodiment.

FIG. 21 shows a result of encapsulating fucoidan using a pen-type structure according to one embodiment.

FIG. 22 shows a result of converting hydrogel into micro-nanoparticles using a pen-type structure according to one embodiment.

FIG. 23 shows a result of various printings using a pen-type structure according to one embodiment.

FIG. 24 shows a result of regenerating tissues using a pen-type structure according to one embodiment.

FIG. 25 shows various embodiments using a pen-type structure according to one embodiment.

[Mode for Invention]

Hereinafter, the present invention will be described in more detail.

The present invention relates to a pen-type structure, that is, a bio-pen, which may be used in the fields of 3D bio-printing, cell therapy carriers, bioactive material carriers, tissue engineering regenerative medicine, medical devices and the like. More particularly, the present invention relates to a pen-type structure for mixing and discharging bio-ink or hydrogel, which is designed to be extruded by homogeneously mixing bio-ink components or hydrogel while minimizing damage to cells.

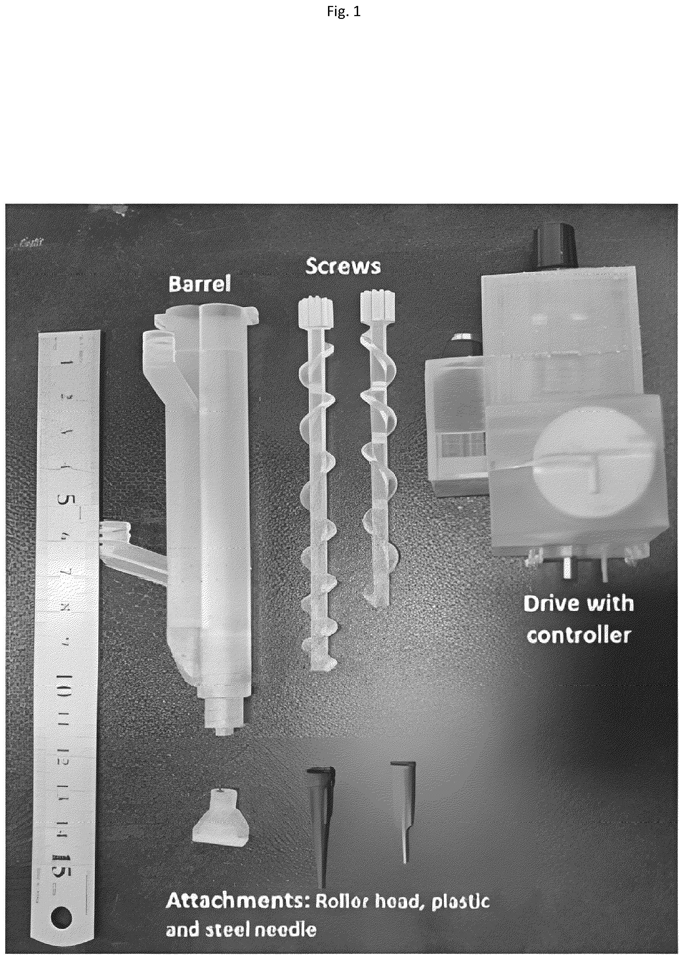

FIG. 1 shows each component of a pen-type structure according to one embodiment.

In one aspect, the present invention may provide a pen-type structure for mixing and discharging a bio-ink or a hydrogel including: a cylindrical first barrel for housing a first screw; a cylindrical second barrel for housing a second screw, which is longer than the first screw and has a structure parallel with the first screw, and formed to have a length longer than that of the first barrel; a controller connected to a gear of the first screw and a gear of the second screw in adjacent to the gear of the first screw and the gear of the second screw so as to drive the first screw and the second screw; two or more supply units formed in the first barrier and supplying a bio-ink or hydrogel material into the first barrel; and a bio-ink or hydrogel discharge unit which extends from an end portion of the second barrel on an opposite side of the controller, and which discharges bio-ink or hydrogel, in which the first barrel and the second barrel are formed to extend in communication with each other so that the first screw and the second screw are not spatially separated, and in which the first screw has a variable pitch of three sections and the second screw has a variable pitch of four sections.

The pen-type structure allows uniform mixing and extrusion of bio-ink or hydrogel including living cells and inanimate materials such as a bioactive material or nanoparticles. In another aspect, the pen-type structure allows uniform mixing and extrusion of inanimate materials such as bioactive materials excluding cells, growth factors, genes, drugs, and nanoparticles.

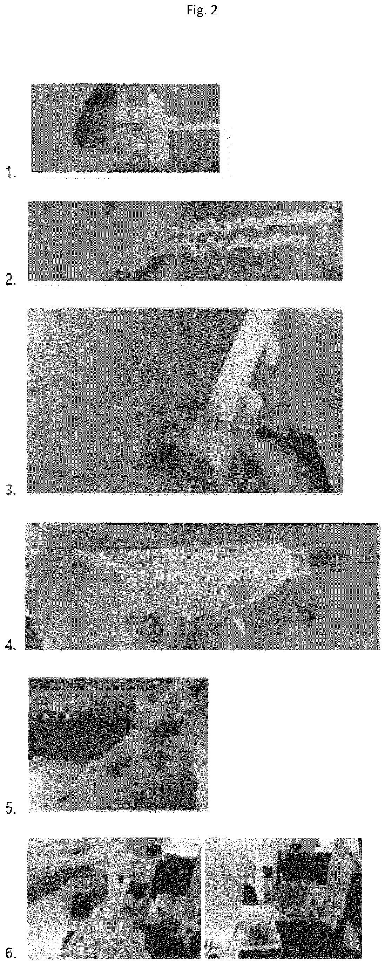

FIG. 2 shows a series of processes for assembling a pen-type structure and mounting on a 3D printer according to one embodiment.

The pen-type structure may be assembled by including a step of attaching a second screw longer than a first screw to a motor shaft, disposing the first screw shorter than the second screw at a phase difference of 90°, housing each screw, fixing an integrated barrel, attaching a printing needle (screw or push type) as an extrusion head to a bio-ink or hydrogel discharge unit, and closing a supply unit with a cap.

The pen-type structure may be used by being directly printed by hand or being mounted on a cradle or a 3D printer.

In one exemplary embodiment, one or more ultraviolet or laser light sources may be attached to the first barrel and/or the second barrel to provide photo crosslinking and/or light irradiation to the bio-ink or hydrogel material, and the light sources may be powered from a power supply of the controller.

FIG. 3 shows a pen-type structure reinforced with a UV-LED irradiation device for optical cross-linking according to one embodiment, that is, a barrel shape of a bio-pen. A tube for preparing a photo-irradiated gel (i.e., a passage for installing an electric wire connected to an upper portion of the barrel and a lower portion of the barrel and a UV LED installation part for installing components on the barrel) and an LED irradiation member (i.e., a UV LED providing part for inducing the activity of the photoinitiator) were added to the barrel. The bio-pen to which the light irradiation device is attached may provide a function of easily progressing crosslinking of bio-ink or hydrogel without the help of an additional UV device.

The pen-type structure according to the present invention may be continuously or semi-continuously batch-mixed and/or 3D-printed with multi-component materials including live cells, gel, nanoparticles or microparticles, bioactive molecules, polymers, cross-linking agents and mixtures thereof, by using a dual-screw extrusion mixing system having different lengths. In addition, when all of the initially loaded bio-ink is used, it may be possible to continuously perform printing by replacing the same with a new bio-ink syringe in the supply unit.

A material of parts such as the first and second screws constituting the pen-type structure according to the present invention may be made of any one of metal, non-metal, and plastic materials, and non-toxic, biocompatible, FDA-approved materials may be used. For example, medical grade steels, plastics, and polymers approved by the FDA may be used. In addition, parts used in a dual-screw extrusion mixing system may be sterilized before use.

The first screw and the second screw may be disposed inside the first barrel and the second barrel, respectively, to mix the materials supplied through the supply unit. The first barrel and the second barrel may be integrally connected.

The controller may be connected to a motor of each of a gear, a belt, or a screw provided in the first and second screws, and may rotate the first and second screws in the same direction or in opposite directions.

In one exemplary embodiment, a speed of the motor of each of the gear, belt, or screw may be adjusted to 0 to 200 rpm or 10 to 200 rpm.

In one exemplary embodiment, the speed of the first and second screws may be achieved via gears, belts or directly from the shaft of the motor.

In one exemplary embodiment, the motor of each of the gear, belt, or screw may be driven through a power supply device of the controller.

In one exemplary embodiment, the power supply device may be a DC power supply device including a power adapter, a direct AC supply, or a USB port of a computer. Motors attached to the first and second screws may be driven through a microcontroller (programmable or non-programmable) provided through a DC power supply device including a power adapter, a direct AC input or a USB port of a computer.

In one exemplary embodiment, the bio-ink materials may include living or non-living materials including living cells, stem cells, gel, nano or micro particles (e.g., bone graft materials, carbon nanotubes, carbon nanofibers, etc.), bioactive molecules (e.g., bone growth factors, cartilage growth factors, blood vessel growth factors, etc.), polymers, cross-linking agents, and mixtures thereof.

In one exemplary embodiment, the first screw and the second screw may have an axial diameter of 0.4 mm to 10 mm or 2 mm to 6 mm and an outer diameter of 0.5 mm to 20 mm or 6 mm to 12 mm.

In one exemplary embodiment, the axial diameter and axial length of the first screw may be 1:4 to 40 or 1:6 to 10. The axial length may mean a length which does not include the gear of the screw.

In one exemplary embodiment, the axial diameter and axial length of the second screw may be 1:4 to 40 or 1:8 to 12. The axial length may mean a length which does not include the gear of the screw.

In one exemplary embodiment, the first screw may have a rectangular cross-sectional shape at a distal end in a direction of the discharge unit, while the second screw may have a conical cross-sectional shape at a distal end in a direction of the discharge unit. In other words, the distal end of the second screw may have a shape in which a diameter of a central axis gradually decreases.

In one exemplary embodiment, the first screw and the second screw may have a lead angle of 0.1° to 60°.

In one exemplary embodiment, the first screw and the second screw may have a screw flange shape with an inclined (0.1° to 60°) structure to push the contents forward.

In one exemplary embodiment, the first screw and the second screw may have a pitch of 2 mm to 50 mm.

In one exemplary embodiment, the first screw and the second screw may have a pitch phase difference of 45° to 135°.

In one exemplary embodiment, the first screw may have a section a, a section b, and a section c sequentially in a direction from the controller to the bio-ink or hydrogel discharge unit, a pitch size of each section may be $b < a \leq c$, the second screw may have a section d, a section e, a section f, and a section g sequentially in a direction from the controller to the bio-ink or hydrogel discharge unit, a pitch size of each section may be $e < d \leq f$, and a pitch size of the section g may satisfy following conditions i) and ii): Accordingly, damage to the injected cells may be minimized.

   i)

$$i)\ g < f$$

   ii)

ii) $e \leq g$ or $e > g$.

A variable pitch of the first screw may sequentially have an initial pitch region for easy transfer from the screw gear (a), a small pitch region for better mixing at high shear rates (b), and a large pitch region for low shear mixing (c), and a length of each area may be changed depending on the requirements. A supply unit for supplying cells may be formed in a large pitch area for low shear mixing. In other words, it may be desirable that materials requiring a low shear process be provided through a barrel in section c.

A variable pitch of the second screw may sequentially have an initial pitch region for easy transfer from the screw gear (d), a small pitch region for better mixing at high shear rates (e), a large pitch region for low shear mixing (f), and a small pitch region in single screw arrangement for uniform extrusion and delivery (g), and a length of each area may be changed depending on the requirements.

The first screw may have a variable pitch, but may have the same height of a thread. The second screw may also have a variable pitch, but may have the same height of a thread.

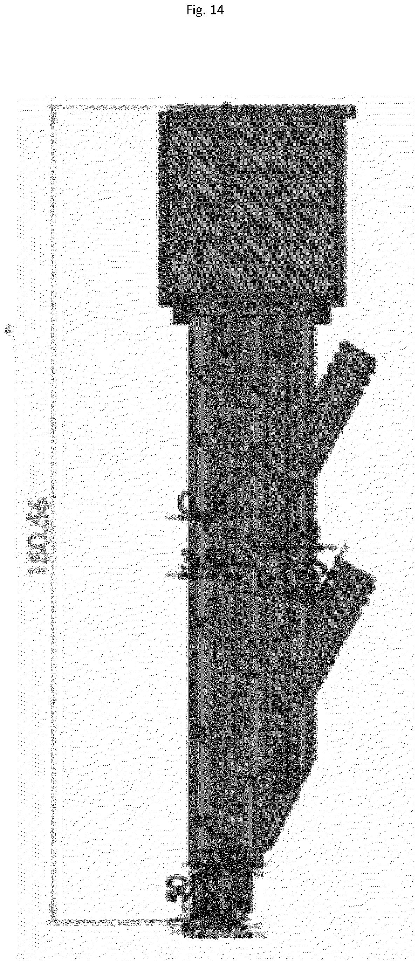

In one exemplary embodiment, a distance between a thread of the first screw and an inner wall of the first barrel may range from 0.005 mm to 0.30 mm, from 0.05 mm to 0.30 mm, or from 0.10 mm to 0.20 mm, and a distance between a thread of the second screw and an inner wall of the second barrel may range from 0.005 mm to 0.30 mm, from 0.05 mm to 0.30 mm, or from 0.10 mm to 0.20 mm (see FIG. 14).

In one exemplary embodiment, a thread of the first screw and a thread of the second screw may be engaged with each other.

In one exemplary embodiment, a distance between a root of the first screw and an inner wall of the first barrel may range from 0.01 mm to 6 mm, from 1 mm to 5 mm, or from 2 mm to 4 mm, and a distance between a root of the second screw and an inner wall of the second barrel may range from 0.01 mm to 6 mm, from 1 mm to 5 mm, or from 2 mm to 4 mm (see FIG. 14).

In one exemplary embodiment, a distance between a shaft center of the first screw and a shaft center of the second screw may be formed to be longer than a shaft diameter of the first screw or the second screw.

In one exemplary embodiment, a distance between a shaft center of the first screw and a shaft center of the second screw may range from 0.5 mm to 20 mm, from 0.5 mm to 12 mm, from 1 mm to 10 mm, from 3 mm to 8 mm, from 0.6 mm to 2.5 mm.

In one exemplary embodiment, the pen-type structure may require backflush to maintain a required gel extrusion pressure and determine a pitch size considering a size of cells, etc.

In one exemplary embodiment, at least one breaker plate of a mesh type may be attached to a delivery surface of the barrel to provide uniform delivery of materials.

Herein, an upper end portion of the first barrel or the second barrel may mean a controller direction, and a lower end portion of the first barrel or the second barrel may mean a direction of the bio-ink or hydrogel discharge unit.

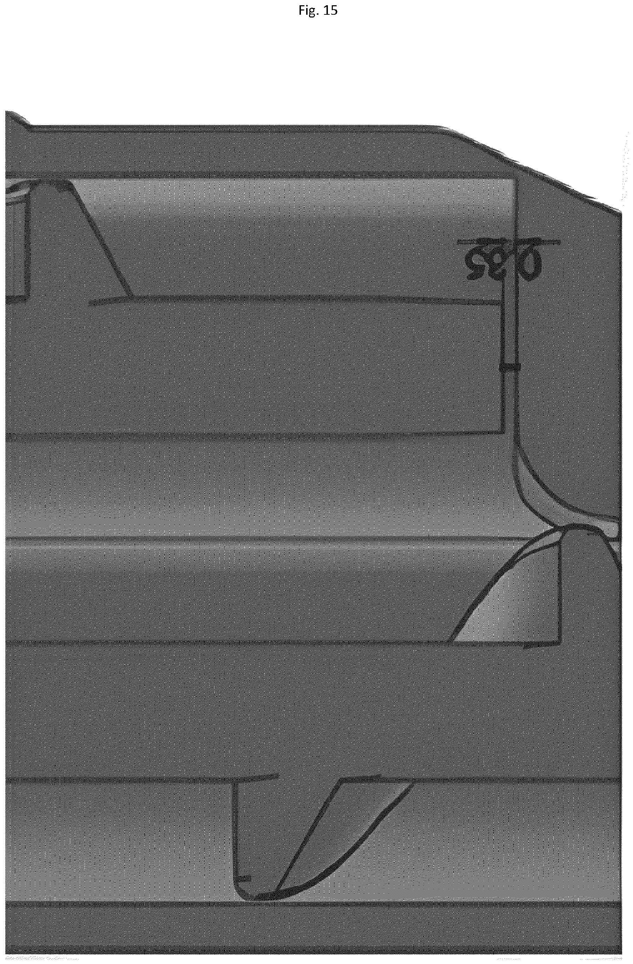

In one exemplary embodiment, a lower end portion of the first barrel may have an inner wall in a direction perpendicular to an axis of the first screw, and a distance between the inner wall and an end point, that is, an end surface of the first screw may range from 0.005 mm to 1 mm, from 0.05 mm to 1 mm, or from 0.1 mm to 0.5 mm (see FIGS. 14 and 15).

In one exemplary embodiment, the second screw may be formed to reach up to a point where a lower end portion of the second barrel and the bio-ink or hydrogel discharge unit come into contact with each other. The pen-type structure may have an effect of extruding gel of high viscosity which may not be extruded with conventional pneumatic or piston-type extrusion systems by precisely and positively controlling the same. In addition, it may be possible to uniformly and finely deposit bio-ink or hydrogel through a precise control and continuously and regularly provide gel and/or bio-ink.

In one exemplary embodiment, the supply unit may supply the bio-ink or hydrogel material at a supply angle of 10° to 90° with respect to the first barrel. In this case, the supply unit may be formed in a region with a small pitch of the first screw and a region with a large pitch of the first screw, respectively.

In one exemplary embodiment, the supply unit may be connected to a syringe (in the form of a screw or in the form of no screw).

In one exemplary embodiment, it may be preferable to inject cells through the supply unit close to the bio-ink or hydrogel discharge unit among the two or more supply units. Among the plurality of supply units, an initial area close to the controller may be an inlet for mixing hydrogel and other additives, while a rear inlet of a low shear area after the high shear area is used for cell injection. Such cell inlet may be used for injection of materials sensitive to bioactivity such as genes, proteins, etc., sensitive to a high shear pressure and a small pitch, thereby suppressing damages.

In one exemplary embodiment, the pen-type structure may use a reverse pitch (reverse rotation) to minimize damage to the injected cells.

In one exemplary embodiment, an interval of the supply units may not exceed a length corresponding to 1/3 of a length of the second barrel.

The bio-ink or hydrogel discharge unit may be designed to be connected to a screw or push type needle, roller, or brush.

In one exemplary embodiment, for large area 3D printing or bio-printing, an extrusion head in the form of a roller having a length of 5 mm to 50 mm and a diameter of 2 mm to 20 mm or an extrusion head in the form of a screen having a length of 5 mm to 50 mm and a hole of a width of 2 mm to 20 mm may be connected to the discharge unit.

In one exemplary embodiment, the bio-ink or hydrogel discharge unit may be formed of any one of a metal material, a non-metal material, and a plastic material.

In one exemplary embodiment, a discharge amount of the bio-ink or hydrogel discharge unit may be 0.1 to 300 mL.

In one exemplary embodiment, the bio-ink or hydrogel discharge unit may be provided with a temperature controller which controls a temperature thereof, and the temperature controller may control a temperature of the bio-ink or hydrogel discharge unit to -50°C to 300°C.

In one exemplary embodiment, the bio-ink or hydrogel discharge unit may be capable of attaching and detaching an extrusion head selected from a roller, a brush, or a needle. Accordingly, there may be an effect in which a large area printing is possible with a 3D printing system capable of printing only a limited area.

In one exemplary embodiment, bio-ink, cells, bioactive particles, or a mixture thereof may be mixed with the pen-type structure, and then the mixed solution may be transferred to a syringe or a bioprinting syringe for use.

In one exemplary embodiment, an injection needle used for 3D printing may be connected to the bio-ink or hydrogel discharge unit.

In one exemplary embodiment, the pen-type structure may be mounted to be operable in a printing system. To attach a piston or pneumatic drive extrusion head to the pen-type structure, a standard fixing head attached to the 3D bio-printer may be used to fix the pen-type structure to the 3D bio-printer.

In one exemplary embodiment, the pen-type structure may have mobility which may be detached from the printing system and operated independently.

Unlike existing 3D bio-printing systems, the pen-type structure according to the present invention may minimize damage to cells, control an extrusion output in a more uniform and precise manner, uniformly mix bio-inks, reduce a load of a driving motor by simplifying screws to one screw at an end of an extruder, and further increase precision by adjusting a diameter of a nozzle of a discharge unit.

In another aspect, the present invention may provide a method for printing bio-ink or hydrogel using the pen-type structure for mixing and discharging the bio-ink or the hydrogel.

In one embodiment, the present invention may provide a method and process for automatically, semi-automatically, or batch mixing multi-component materials including living cells, gels, nano/micro particles, bioactive materials, polymers, cross-linkers, or mixtures thereof, and then manually or automatically applying to an irregular part such as soft, flat, uneven, and different-sized areas according to height of an organism or an inanimate matter for various tissue regeneration in the field of musculoskeletal system, dental, ophthalmology, circulatory instruments, etc.

In one embodiment, the present invention may provide a method for automatically, semi-automatically, or batch mixing multi-component materials including polymers, gel, nano/micro particles, drugs, bioactive materials, or mixtures thereof, in order to encapsulate the bioactive materials or drugs in a polymer or gel matrix capable of sustained release delivery of the encapsulated bioactive materials or drugs into a target site.

In one embodiment, the present invention may provide a method for automatically, semi-automatically or batch mixing multi-component materials including polymers, gel, nano/micro particles, drugs, bioactive materials or mixtures thereof, in order to prepare polymer nano- or micro particles which are encapsulated or not with bioactive materials or drugs.

In one embodiment, the method for printing bio-ink or hydrogel may include: sterilizing screws, barrels, and other components with high temperature-high pressure sterilization, ethanol, and/or ultraviolet rays; assembling the screws, barrels, and other components and then injecting a polymer solution, gel, drug, nanoparticles, etc., into an upper supply unit; rotating the screws at a low rpm during the injection; closing the supply unit after the injection; injecting cells using a lower supply unit; closing both an upper supply unit and a lower supply unit before starting extrusion; and setting a screw rpm and an extrusion time to a desired level to extrude bio-ink or hydrogel.

In one embodiment, it may include closing both the upper supply unit and the lower supply unit and then irradiating UV light if necessary.

In one embodiment, the extruding of bio-ink or hydrogel may include connecting a roller, a brush, etc., to perform printing at a large area or printing with lamination.

In one embodiment, it may further include a method for mixing the bio-ink, cells, bioactive particles, or the like, and then transferring the resulting mixture to a syringe, a bio-printing nozzle, or the like for use.

In one embodiment, it may be possible to load a prefabricated gel formed using the pen type structure of the present invention, for example, a drug such as fucoidan, an injectable agent (e.g., corticosteroid, hyaluronic acid, etc.) in joints, an osteoarthritis therapeutic agent, a spinal disease therapeutic agent, a vascular disease therapeutic agent, a tissue regeneration promoter (e.g., bone morphogenic proteins), a low molecular weight drug, oligopeptide, protein, nucleic acid, a bionew drug, a biosimilar drug, a growth factor, and the like as a low molecular weight (LMW, 3-10 kDa) and high molecular weight (HMW, 150-200 kD) model drug. A hydrogel in which a drug is encapsulated may be prepared by operating the pen-type structure to expand and recover a gel network using a variable pitch such that the drug is not destroyed until the drug reaches a destination and by encapsulating the drug in the formed gel.

As another example, after the discharge unit of the pen-type structure is closed, the operation of the pen-type structure may be repeated several times to cut the gel network encapsulated with the drug, thereby miniaturizing the gel to prepare the nanoparticles (NPs) gel. Four variable pitches of the second screw used at this time may be more efficient in preparing a nanoparticle gel. In other words, nanoparticles may be more successfully formed due to a high screw rpm, an increased residence time, and the increased number of variable pitch areas.

As described above, the pen-type structure according to the present invention may provide a high rate of drug encapsulation. For example, in loading a high molecular weight drug such as protein, fucoidan, hyaluronic acid, or nucleic acid in addition to a low molecular weight drug such as tetracycline or corticosteroid (triamcinolone, etc.) into a crosslinked hydrogel, a loss of drug may be minimized. In addition, encapsulation efficiency may be increased and bio-ink or hydrogel components may be uniformly mixed and subjected to sustained release to be applied to drug delivery through a gel and/or nanoparticle gel.

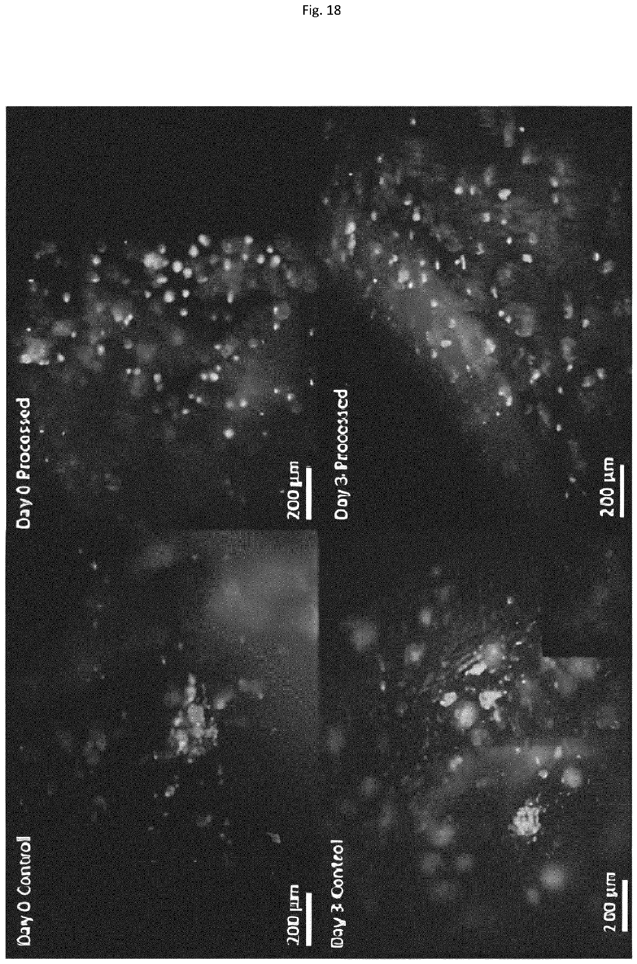

FIG. 18 shows a result of observing bio-ink with a fluorescence microscope immediately after mixing a bio-ink material using a pen-type structure according to one embodiment and after culturing *in vitro* cells for three days. A control group (a bio-ink in which cells and gel are mixed using a spatula) and an experimental group (a bio-ink in which cells and gel are mixed at 15 rpm using the pen-type structure of the present invention) were compared with each other, and a uniform dispersion of the cells in the experimental group could be confirmed.

In one embodiment, in the case of the pen-type structure according to the present invention, it may be possible to add nanoclay particles (e.g., kaolin, bioglass, calcium triphosphate, etc.) having various concentrations for mixing and subsequent extrusion of nanoparticles, for example, bio-ink components including live cells. Shear mixing of different components of bio-ink or hydrogel using such pen-type structure may be effective for homogeneous distribution and extrusion of nanoclay particles and living cells. As a result, it was found that a cyclic compressive load capacity is increased at least four times, and a cell proliferative capacity is improved almost four times in three days.

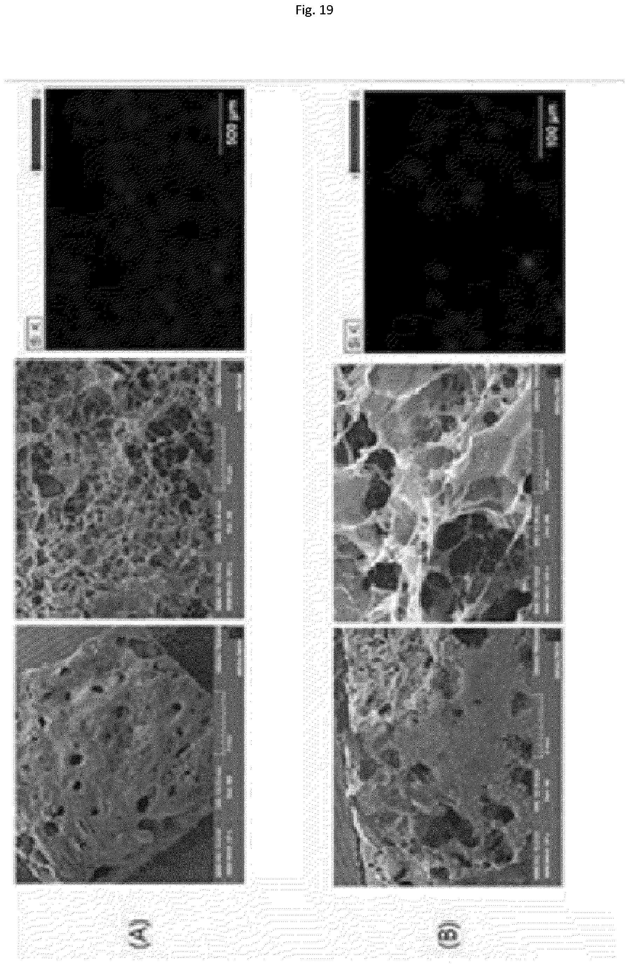

FIG. 19 shows a comparison between an electron microscope observation result and an energy-dispersive X-ray spectroscopy (EDS) observation result for a result obtained by directly printing an alginate-chitosan-kaolin composite gel on a roller after (A) mixing with the pen-type structure according to one embodiment and (B) mixing with a pipette. (A) shows a uniform dispersion of kaolin by mixing an alginate-chitosan-4% kaolin nanoclay hydrogel with the pen-type structure and then printing, and (B) shows a non-uniform distribution and aggregation of kaolin by mixing an alginate-chitosan-4% kaolin nanoclay hydrogel with a pipette and then printing. In the result of mixing with the pen-type structure, the formation of more uniform and small pores was confirmed.

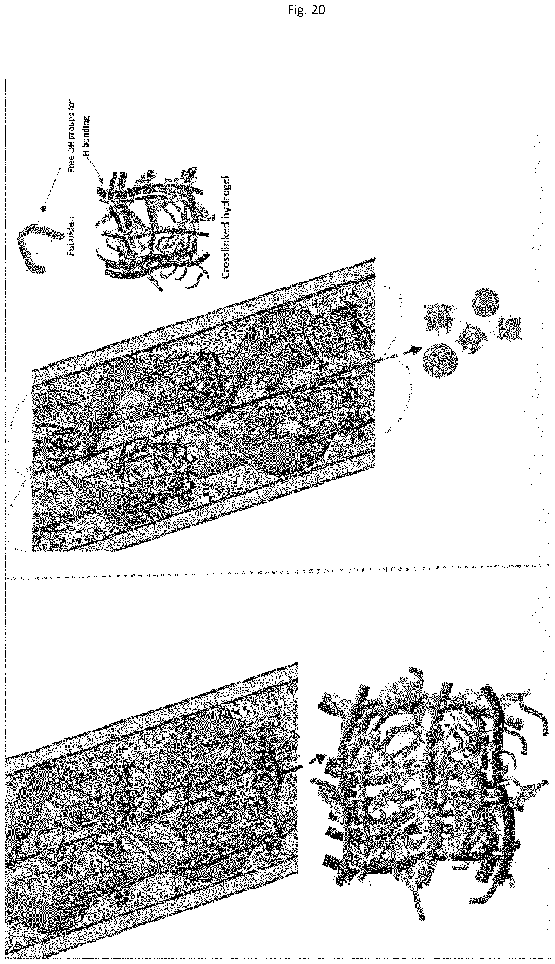

FIG. 20 is a schematic view showing a mechanism of loading a high molecular weight model drug fucoidan into hydrogel using a pen-type structure and a method for preparing micro- or nanogel particles according to one embodiment. A schematic view on the left shows that two screws induce high shear mixing and fucoidan is loaded into a gel structure, while a schematic view on the right shows a recirculation to produce gel nanoparticles with the fucoidan encapsulated.

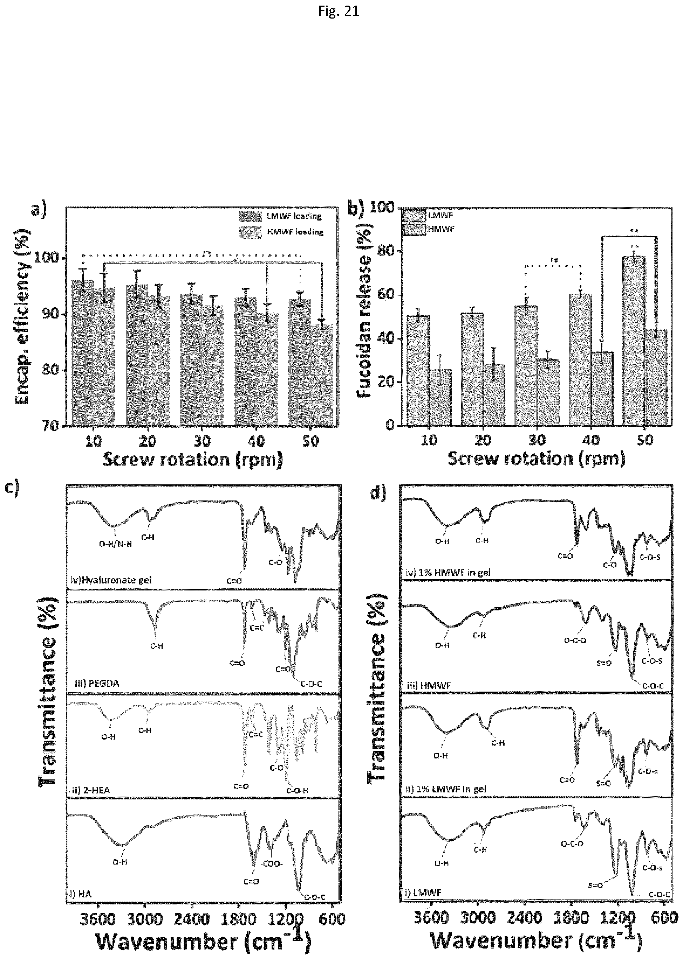

FIG. 21 shows (a) efficiency of loading a model drug fucoidan (low molecular weight, high molecular weight) into hydrogel according to a speed of screw rotation using the pen-type structure according to one embodiment, (b) a release behavior of the loaded fucoidan, and (c, d) results of fourier transform infrared spectroscopy (FTIR) for hydrogel components (hyaluronic acid-hydroxyethyl acrylate-polyethylene glycol diacrylate, fucoidan). It has been shown that drug encapsulation efficiency may be controlled by observing that the encapsulation of the drug is efficient at a specific rpm (e.g., more efficient at 20 rpm rather than at a high rpm).

(a) of FIG. 21 shows that when the rpm is set to 10-50, the efficiency of loading fucoidan into the prepared hydrogel is 89-97%, and the low molecular weight fucoidan is more efficient than the high molecular weight fucoidan. In one experiment according to one embodiment of the present invention, the experiment was performed with nanoparticles of a bioactive material (drug, fucoidan) and drug containing efficiency rather than cell damage. In addition, in the experiment, the discharge unit was blocked and the screw was repeatedly operated (backflush) when the hydrogel was induced into nanoparticles, and then it was confirmed that the gel is prepared in a size of about 20 nm while the gels are raised and lowered again by the backflush to induce a particle size. In the case of the backflush, the loading of cells and fucoidan using the pen-type structure of the present invention and the 3D bioprinting show good results in the efficiency of encapsulating cells and drug, and it is shown that the backflush performed in hydrogel or bio-ink printing with the outlet closed is used as an advantageous technique to prepare the molded gel into nanogels. However, in a normal case in which continuous printing is to be performed while loading a drug and/or cells (in a case in which drug encapsulation and printing into bio-ink or gel containing cells and/or a bioactive material are to be continuously performed), a backflush function need not to be used in order to prevent damage to the cells and gel network while the discharge unit is blocked. In other words, the screw needs to be continuously operated so that the gel (bio-ink) containing cells is printed. The screw of the present invention may be a complex form of a twin screw, and the second screw may be configured as a four-shear zone so that the backflush efficiency may be more efficient than a three-shear zone. In the four-shear zone system, there may be an effect of preparing nano-particles while three times of backflushing occurs.

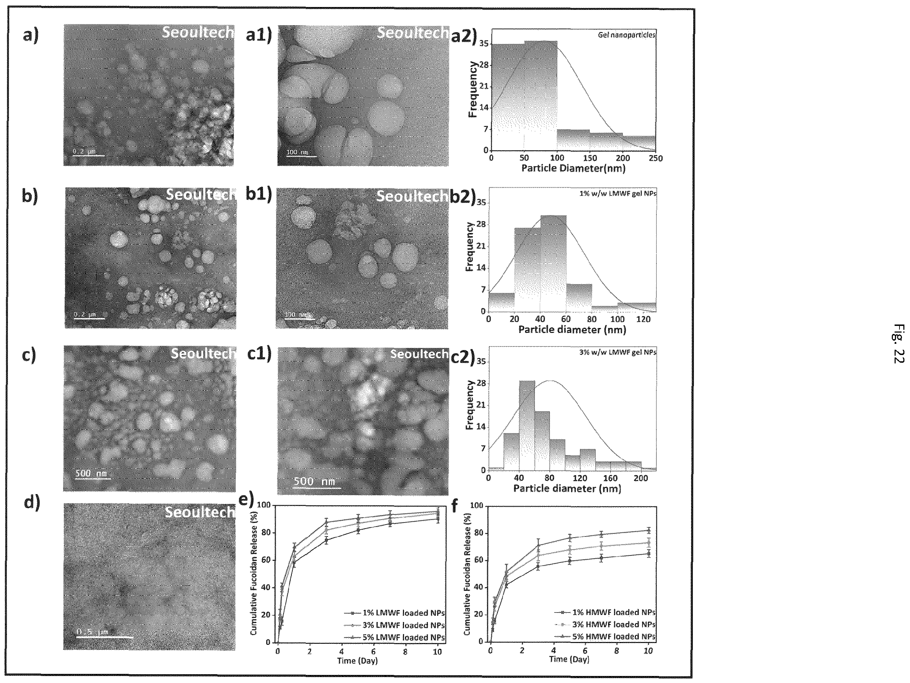

FIG. 22 shows electron micrographs (a, a1, b, b1, c, c1) obtained by converting crosslinked hydrogel into micro- and/or nano-particles by repeatedly rotating a pen-type structure according to one embodiment, particle distributions (a2, b2, c2), a powder-shaped dry state (d), and low molecular weight and high molecular weight fucoidan release behaviors (e, f) loaded inside gel.

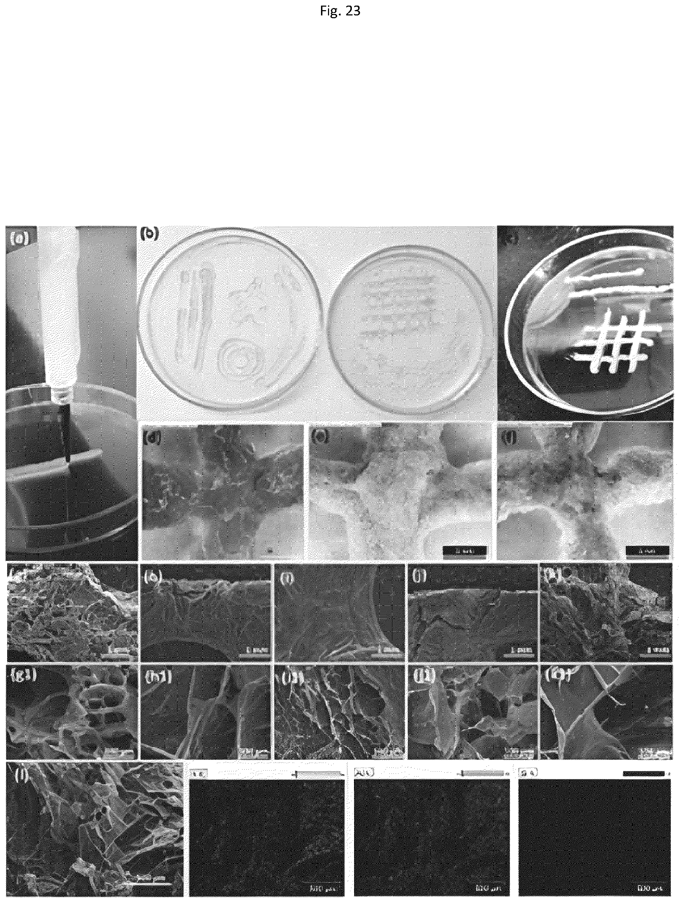

FIG. 23 shows various printing states (a-c) using a pen-type structure according to one embodiment, gel printing states (d-f) at an intersection point, electron microscope photographs (g-l), and an EDS mapping.

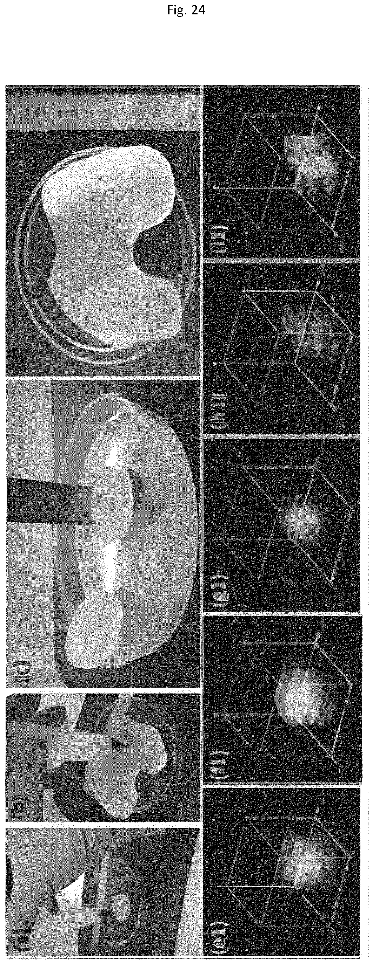

FIG. 24 shows cell viability and tissue regeneration of a regenerated tissue layer (e1-i1) observed after laminating and printing a gel containing cells in three-to-five layers on a bone-cartilage complex tissue defect model (a-d) using a pen-type structure according to one embodiment.

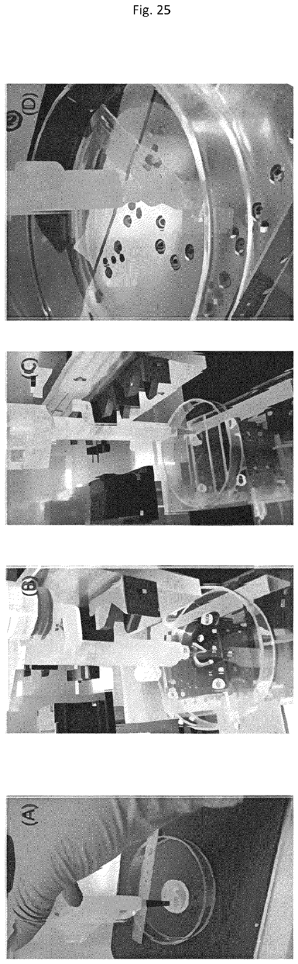

FIG. 25 shows an example of using a pen-type structure according to one embodiment, that is, (A) a process of directly printing inside a complicated shape of a bone-cartilage composite tissue defect model, (B) a process of printing using a metal needle by mounting the pen-type structure on a 3D printer, (C) a process of line printing using a plastic needle, and (D) a process of large-area lamination printing using a roller.

While specific portions of the present invention have been described in detail above, it is apparent to those skilled in the art that such detailed descriptions are set forth to illustrate exemplary embodiments only, but are not construed to limit the scope of the present invention. Thus, it should be understood that the substantial scope of the present invention is defined by the accompanying claims and equivalents thereto.

**Claims**

1. A pen-type structure for mixing and discharging bio-ink or hydrogel comprising:

   a cylindrical first barrel configured to housing a first screw;
   a cylindrical second barrel for housing a second screw, which is longer than the first screw and has a structure parallel with the first screw, and formed to have a length longer than a length of the first barrel;
   a controller connected to a gear of the first screw and a gear of the second screw in adjacent to the gear of the first screw and the gear of the second screw so as to drive the first screw and the second screw;
   two or more supply units formed in the first barrel and supplying a bio-ink or hydrogel material into the first barrel; and
   a bio-ink or hydrogel discharge unit which extends from an end portion of the second barrel on an opposite side of the controller, and which discharges bio-ink or hydrogel,
   wherein the first barrel and the second barrel are formed to extend in communication with each other so that the first screw and the second screw are not spatially separated, and
   wherein the first screw has a variable pitch of three sections and the second screw has a variable pitch of four sections.

2. The pen-type structure of claim 1, wherein the first screw has a section a, a section b, and a section c sequentially in a direction from the controller to the bio-ink or hydrogel discharge unit, a pitch size of each section is $b < a \leq c$, the second screw has a section d, a section e, a section f, and a section g sequentially in a direction from the controller to the bio-ink or hydrogel discharge unit, a pitch size of each section is $e < d \leq f$, and a pitch size of the section g satisfies following conditions i) and ii):

   i)

   $$i)\ g < f$$

   ii)

   $$ii)\ e \leq g\ or\ e > g.$$

3. The pen-type structure of claim 1, wherein the first screw and the second screw have a pitch phase difference of 45° to 135°.

4. The pen-type structure of claim 1, wherein a distance between a thread of the first screw and an inner wall of the first barrel ranges from 0.005 mm to 0.30 mm, and a distance between a thread of the second screw and an inner wall of the second barrel ranges from 0.005 mm to 0.30 mm.

5. The pen-type structure of claim 1, wherein a distance between a root of the first screw and an inner wall of the first barrel ranges from 0.01 mm to 6 mm, and a distance between a root of the second screw and an inner wall of the second barrel ranges from 0.01 mm to 6 mm.

6. The pen-type structure of claim 1, wherein a distance between a shaft center of the first screw and a shaft center of the second screw is formed to be longer than a shaft diameter of the first screw or the second screw.

7. The pen-type structure of claim 1, wherein a lower end portion of the first barrel has an inner wall in a direction perpendicular to an axis of the first screw, and a distance between the inner wall and an end point of the first screw ranges from 0.005 mm to 1 mm.

8. The pen-type structure of claim 1, wherein the second screw is formed up to a point where a lower end portion of the second barrel and the bio-ink discharge unit come into contact with each other.

9. The pen-type structure of claim 1, wherein an interval of the supply units does not exceed a length corresponding to 1/3 of a length of the second barrel.

10. The pen-type structure of claim 1, wherein the bio-ink discharge unit is capable of attaching and detaching an extrusion head selected from a roller, a brush, or a needle.

11. The pen-type structure of claim 1, wherein the first barrel and/or the second barrel have one or more ultraviolet or laser light sources so that light is irradiated to the bio-ink or hydrogel to be discharged.

12. The pen-type structure of claim 1, wherein the pen-type structure is mounted to be operable in a printing system.

13. A method for printing bio-ink or hydrogel using a pen-type structure for mixing and discharging bio-ink or hydrogel according to any one of claims 1 to 12.

14. The method of claim 13, further comprising: preparing nanogel from shaped hydrogel.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Micro USB power port for backup power supply

Battery case for 3Volt coin cell battery(3기|)

Controller output connected to RPM controller:
NC – normally closed
NO – normally open
COM – negative (-)

Power supply
6-30 volt DC
(+)Ground (-)

Program buttons

Power adapter port

**ON/OFF switch and RPM controller**

Fig. 18

Fig. 19

(A)

(B)

EP 4 446 093 A1

Fig. 20

Fig. 21

EP 4 446 093 A1

Fig. 22

Fig. 23

Fig. 24

Fig. 25

## INTERNATIONAL SEARCH REPORT

| International application No. |
|---|
| **PCT/KR2022/019702** |

| A. | CLASSIFICATION OF SUBJECT MATTER |
|---|---|

**B29C 64/209**(2017.01)i; **C12M 1/00**(2006.01)i; **B29C 64/321**(2017.01)i; **B29C 48/30**(2019.01)i; **B29C 48/40**(2019.01)i; **B29C 48/285**(2019.01)i; **B29C 48/68**(2019.01)i; **B33Y 30/00**(2015.01)i; **B33Y 40/00**(2015.01)i; **B33Y 70/00**(2015.01)i

According to International Patent Classification (IPC) or to both national classification and IPC

| B. | FIELDS SEARCHED |
|---|---|

Minimum documentation searched (classification system followed by classification symbols)

B29C 64/209(2017.01); B29B 7/46(2006.01); B29C 47/40(2006.01); B29C 47/60(2006.01); B29C 48/40(2019.01); B29C 48/67(2019.01); B29C 64/118(2017.01); B29C 64/20(2017.01); B33Y 40/00(2015.01); B33Y 70/10(2020.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 펜(pen), 프린팅(printing), 스크류(screw), 바이오잉크(bio ink), 하이드로젤 (hydrogel)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2286073 B1 (FOUNDATION FOR RESEARCH AND BUSINESS, SEOUL NATIONAL UNIVERSITY OF SCIENCE AND TECHNOLOGY) 06 August 2021 (2021-08-06)<br>  See claims 1-10; paragraphs [0038]-[0040] and [0060]; and figures 1-4. | 1-14 |
| Y | CN 110103444 A (SHANGHAI JINHU EXTRUSION EQUIPMENT CO., LTD.) 09 August 2019 (2019-08-09)<br>  See claims 1-2; and figure 2. | 1-14 |
| A | CN 206884174 U (JIANGMEN CITY MANGO 3 D-PRINTING SCIENCE AND TECHNOLOGY) 16 January 2018 (2018-01-16)<br>  See claim 1; and figures 1-2. | 1-14 |
| A | JP 2004-181954 A (CALP CORP.) 02 July 2004 (2004-07-02)<br>  See paragraph [0045]; and figure 1. | 1-14 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **14 March 2023** | **15 March 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2022/019702** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 06-055612 A (KOBE STEEL LTD.) 01 March 1994 (1994-03-01)<br>See entire document. | 1-14 |

Form PCT/ISA/210 (second sheet) (July 2022)

**INTERNATIONAL SEARCH REPORT**
Information on patent family members

International application No.

**PCT/KR2022/019702**

| Patent document cited in search report | | | Publication date (day/month/year) | Patent family member(s) | | | Publication date (day/month/year) |
|---|---|---|---|---|---|---|---|
| KR | 10-2286073 | B1 | 06 August 2021 | None | | | |
| CN | 110103444 | A | 09 August 2019 | None | | | |
| CN | 206884174 | U | 16 January 2018 | None | | | |
| JP | 2004-181954 | A | 02 July 2004 | JP | 2008-181954 | A5 | 17 January 2008 |
| | | | | JP | 4303570 | B2 | 29 July 2009 |
| JP | 06-055612 | A | 01 March 1994 | None | | | |

Form PCT/ISA/210 (patent family annex) (July 2022)

**EP 4 446 093 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 102286073